# EUROPÄISCHE PATENTSCHRIFT

(11) **EP 0 776 653 B1**
(45) Veröffentlichungstag und Bekanntmachung des Hinweises auf die Patenterteilung: **20.03.2002**
(21) Anmeldenummer: 96118746.5
(22) Anmeldetag: 22.11.1996
(51) Int. Cl.: A61K 7/48

(54) **Polyhydroxyfettsäureamide enthaltende kosmetische und/oder pharmazeutische Zubereitungen**
Cosmetic and/or pharmaceutical compositions containing polyhydroxy fatty acid amides
Compositions cosmétiques et/ou pharmaceutiques contenant des amides d'acides gras polyhydroxyle

(30) Priorität: 30.11.1995 DE 19544710
(43) Veröffentlichungstag der Anmeldung: 04.06.1997
(73) Patentinhaber: Cognis Deutschland GmbH, 40589 Düsseldorf (DE)
(72) Erfinder: Seipel, Werner, 40723 Hilden (DE); Fabry, Bernd, Dr., 41352 Korschenbroich (DE); Boyxen, Norbert, 47906 Kempen (DE); Rottmann, Mirella, 40231 Düsseldorf (DE)

(56) Entgegenhaltungen:
- EP-A- 0 450 527
- WO-A-95/14658
- DE-A- 4 309 567
- DE-A- 4 326 959
- DATABASE WPI Week 8016 Derwent Publications Ltd., London, GB; AN 80-28435c XP002027372 "Water-in-oil emulsion based skin cream - contains fatty acid alkanol-amide and polyol-fatty acid ester surfactant" & JP 55 033 447 A (KANEBO) , 9.März 1980

## Beschreibung

### Gebiet der Erfindung

Die Erfindung betrifft kosmetische und/oder pharmazeutische Zubereitungen mit einem Gehalt an Fettsäure-N-alkylpolyhydroxyalkylamiden und Oligoglycerinen bzw. deren Estern mit Fettsäuren.

### Stand der Technik

Zubereitungen, die zur Reinigung und Pflege der menschlichen Haut und der Haare eingesetzt werden, enthalten in der Regel ein oder mehrere oberflächenaktive Substanzen, insbesondere auf Basis von anionischen oder amphoteren Tensiden. Da die alleinige Verwendung von Tensiden Haut und Haare zu sehr austrocknen würde, ist es im allgemeinen üblich, solchen Mitteln rückfettende Substanzen zuzusetzen. Es liegt dabei auf der Hand, daß diese Stoffe nicht nur eine hinreichende rückfettende Wirkung, sondern den Erfordernissen des Marktes folgend gleichzeitig auch eine optimale dermatologische Verträglichkeit aufweisen müssen.

Die Aufgabe der Erfindung hat somit darin bestanden, ethylenoxidfreie Rückfettungsmittel zur Verfügung zu stellen, die bei Raumtemperatur flüssig sind, verdickende Eigenschaften aufweisen und insbesondere eine besonders hohe dermatologische Verträglichkeit aufweisen.

### Beschreibung der Erfindung

Gegenstand der Erfindung sind kosmetische und/oder pharmazeutische Zubereitungen, enthaltend
(a) Fettsäure-N-alkylpolyhydroxyalkylamide und
(b1) Oligoglycerine und/oder
(b2) Oligoglycerinfettsäureester.

Überraschenderweise wurde gefunden, daß Mischungen der genannten Fettsäureamide mit Oligoglycerinen bzw. Oligoglycerinestern eine besonders hohe dermatologische Verträglichkeit bei optimalen rückfettenden Eigenschaften aufweisen. Die Mischungen sind zudem bei Raumtemperatur flüssig, so daß sie sich auch kalt verarbeiten lassen. Ein weiterer Vorteil besteht darin, daß die Mischungen in tensidischen Systemen eine Viskosität aufbauen.

### Fettsäure-N-alkylpolyhydroxyalkylamide

Fettsäure-N-alkylpolyhydroxyalkylamide stellen nichtionische Tenside dar, die der Formel **(I)** folgen, 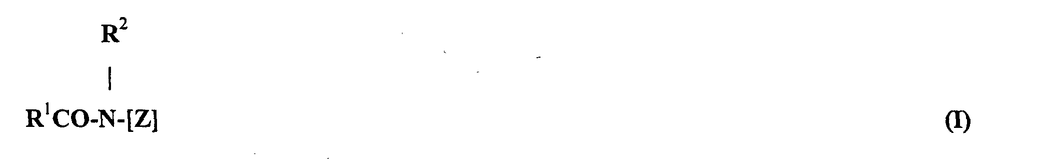 in der R¹CO für einen aliphatischen Acylrest mit 6 bis 22 Kohlenstoffatomen, R² für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften **US 1,985,424, US 2,016,962** und **US 2,703,798** sowie die Internationale Patentanmeldung **WO 92/06984** verwiesen. Eine Übersicht zu diesem Thema von H.Kelkenberg findet sich in **Tens. Surf. Det. 25, 8 (1988)**.

Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher **Fettsäure-N-alkylglucamide** dar, wie sie durch die Formel **(II)** wiedergegeben werden:

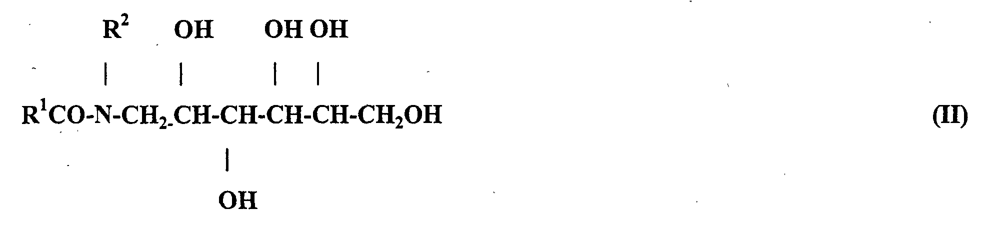

Vorzugsweise werden als Fettsäure-N-alkylpolyhydroxyalkylamide Glucamide der Formel **(II)** eingesetzt, in der R² für Wasserstoff oder eine Alkylgruppe steht und R¹CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkyl-glucamide der Formel **(II)**, die durch reduktive Aminierung von Glucose mit Methylamin und anschließende Acylierung mit Laurinsäure oder C_{12/14}-Kokosfettsäure bzw. einem entsprechenden Derivat erhalten werden. Weiterhin können sich die Polyhydroxyalkylamide auch von Maltose und Palatinose ableiten.

Auch die Verwendung der Fettsäure-N-alkylpolyhydroxyalkylamide ist Gegenstand einer Vielzahl von Veröffentlichungen. Aus der Europäischen Patentanmeldung **EP-A1 0 285 768** (Hüls) ist beispielsweise ihr Einsatz als Verdickungsmittel bekannt. In der Französischen Offenlegungsschrift **FR-A 1 580 491** (Henkel) werden wäßrige Detergensgemische auf Basis von Sulfaten und/oder Sulfonaten, Niotensiden und gegebenenfalls Seifen beschrieben, die Fettsäure-N-alkylglucamide als Schaumregulatoren enthalten. Mischungen von kurz- und längerkettigen Glucamiden werden in der Deutschen Patentschrift **DE-C1 44 00 632** (Henkel) beschrieben. In den Deutschen Offenlegungsschriften **DE-A1 42 36 958** und **DE-A1 43 09 567** (Henkel) wird ferner über den Einsatz von Glucamiden mit längeren Alkylresten als Pseudoceramide in Hautpflegemitteln sowie über Kombinationen von Glucamiden mit Proteinhydrolysaten und kationischen Tensiden in Haarpflegeprodukten berichtet. Gegenstand der Internationalen Patentanmeldungen **WO 92/06153, WO 92/06156, WO 92/06157, WO 92/06158, WO 92/06159** und **WO 92/06160** (Procter & Gamble) sind Mischungen von Fettsäure-N-alkyl-glucamiden mit anionischen Tensiden, Tensiden mit Sulfat- und/oder Sulfonatstruktur, Ethercarbonsäuren, Ethersulfaten, Methylestersulfonaten und nichtionischen Tensiden. Die Verwendung dieser Stoffe in den unterschiedlichsten Wasch-, Spül- und Reinigungsmitteln wird in den Internationalen Patentanmeldungen **WO 92/06152, WO 92/06154, WO 92/06155, WO 92/06161, WO 92/06162, WO 92/06164, WO 92/06170, WO 92/06171** und **WO 92/06172** (Procter & Gamble) beschrieben. Der Anteil der Amide an den Zubereitungen beträgt üblicherweise 0,5 bis 70, vorzugsweise 1 bis 65 und insbesondere 20 bis 50 Gew.-%.

### Oligoglycerine

Oligoglycerine, die vielfach nicht ganz zutreffend auch als Polyglycerine bezeichnet werden, stellen bekannte Stoffe dar, die nach den einschlägigen Verfahren der präparativen organischen Chemie erhalten werden können. Üblicherweise geht man dabei von Glycerin aus, das in Gegenwart basischer Katalysatoren wie beispielsweise Natriumhydroxid, Alkalisilicaten, Alkaliboraten oder Zeolithen bei Temperaturen im Bereich von 180 bis 250°C kondensiert wird. Die dabei anfallenden technischen Gemische weisen in der Regel einen mittleren Eigenkondensatonsgrad im Bereich von 1,1 bis 10 und vorzugsweise 1,5 bis 5 auf. Neben freiem Glycerin sind vor allem Diglycerin und Triglycerin enthalten, wobei das Verhältnis dieser Komponenten zueinander im wesentlichen durch den Katalysator bestimmt wird. Ein typisches Oligoglycerin weist beispielsweise die folgende Zusammensetzung auf:

| | |
|---|---|
| Glycerin | 1 bis 10 Gew.-% |
| Diglycerin | 15 bis 45 Gew.-% |
| Triglycerin | 25 bis 35 Gew.-% |
| Tetraglycerin | 10 bis 20 Gew.-% |
| Pentaglycerin | 5 bis 15 Gew.-% |
| Hexaglycerin | 3 bis 10 Gew.-% |
| Oligoglycerine | ad 100 Gew.-% |

Der Anteil der Oligoglycerine an den Zubereitungen beträgt üblicherweise 0,5 bis 40, vorzugsweise 1 bis 35 und insbesondere 20 bis 30 Gew.-%.

### Oligoglycerinfettsäureester

Auch Oligoglycerinfettsäureester stellen bekannte Stoffe dar, die man üblicherweise durch Veresterung von Oligoglycerinen mit Fettsäuren herstellt. Bevorzugte Ester leiten sich von den oben genannten technischen Oligoglyceringemischen ab, die einen mittleren Eigenkondensationsgrad im Bereich von 1,1 bis 5 aufweisen. Die Fettsäurekomponente kann sich von Fettsäuren der Formel **(III)** ableiten,

**R**^{**3**}**CO-OH** (III)

in der R³CO für einen linearen oder verzweigten Acylrest mit 6 bis 22 Kohlenstoffatomen und 0 und/oder 1, 2 oder 3 Doppelbindungen steht.

Typische Beispiele sind Ester von technischen Oligoglycerinen mit Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen. Bevorzugt sind Ester von Oligoglycerinen mit einem mittleren Eigenkondensationsgrad im Bereich von 1,1 bis 5 und insbesondere 1,5 bis 4 mit technischen Fettsäuren mit 12 bis 18 Kohlenstoffatomen wie beispielsweise Kokosfettsäure oder Isostearinsäure. Die Ester können zudem pro Mol Oligoglycerinkomponente 1 bis 25 und vorzugsweise 5 bis 15 Mol Ethylenoxid enthalten. Die ethoxylierten Ester sind erhältlich, indem man entweder ethoxylierte Oligoglycerine verestert oder die Ethylenoxideinheiten nachträglich durch Insertion in die Esterbindung in das Molekül einbaut. Die Oligoglycerinfettsäureester weisen in der Regel einen Veresterungsgrad im Bereich von 1 bis 3 und vorzugsweise 1,5 bis 2,5 auf. Der Anteil der Oligoglycerinester an den Zubereitungen beträgt üblicherweise 0,5 bis 40, vorzugsweise 1 bis 35 und insbesondere 20 bis 30 Gew.-%.

### Tenside

Die erfindungsgemäßen Zubereitungen können 0,5 bis 40 und vorzugsweise 1 bis 25 Gew.-% anionische und/oder amphotere bzw. zwitterionische Tenside enthalten. Typische Beispiele für **anionische Tenside** sind Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, Acyllactylate, Acyltartrate, Acylglutamate, Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124** oder **J.Falbe (ed.), "Katalysatoren, Tenside und Mineralöladditive", Thieme Verlag, Stuttgart, 1978, S. 123-217** verwiesen.

### Ölkörper

Die erfindungsgemäßen Zubereitungen können 0,5 bis 25 und vorzugsweise 1 bis 15 Gew.-% Ölkörper enthalten. Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₁₈-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimerdiol) und/oder Guerbetalkoholen, Triglyceride auf Basis C₆-C₁₀-Fettsäuren, pflanzliche Öle, verzweigte primäre Alkohole, substituierte Cyclohexane, Guerbetcarbonate, Dialkylether und/oder aliphatische bzw. naphthenische Kohlenwasserstoffe in Betracht.

### Zubereitungen

Eine typische rückfettende Zubereitung, die den Gegenstand der Erfindung illustrieren soll, kann beispielsweise eine Zusammensetzung gemäß Tabelle 1 aufweisen:

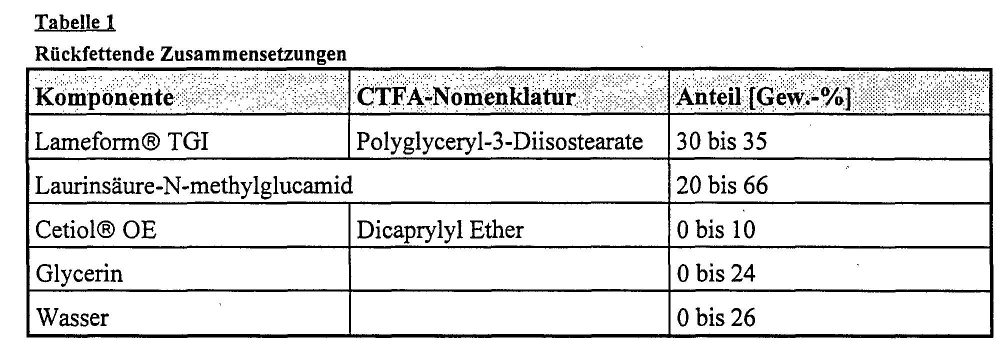

Demzufolge setzen sich besonders vorteilhafte Zubereitungen wie folgt zusammen:
(a) 0,5 bis 70 Gew.-% Fettsäure-N-alkylpolyhydroxyalkylamide,
(b) 0,5 bis 40 Gew.-% Oligoglycerine und/oder Oligoglycerinfettsäureester,
(c) 0 bis 40 Gew.-% anionische, nichtionische, kationische und/oder amphotere bzw. zwitterionische Tenside,
(d) 0 bis 25 Gew.-% Ölkörper und
(e) 0 bis 25 Gew.-% Glycerin und/oder Wasser.

Falls erforderlich, können die erfindungsgemäßen Zubereitungen jeweils 0,5 bis 25 und vorzugsweise 1 bis 15 Gew.-% Glycerin und/oder Wasser enthalten.

### Gewerbliche Anwendbarkeit

Die erfindungsgemäßen Zubereitungen zeichnen sich durch eine hohe dermatologische Verträglichkeit und ausgezeichnete rückfettende Eigenschaften aus. Sie sind flüssig und pumpbar und lassen sich daher kalt verarbeiten. Sie sind frei von Ethylenoxid und auch in Abwesenheit von Konservierungsmitteln gegen mikrobiellen Befall ausreichend stabilisiert. Sie weisen verdickende Eigenschaften auf und sind dabei vollständig biologisch abbaubar.

Ein weiterer Gegenstand der Erfindung betrifft daher ihre Verwendung als Rückfettungsmittel zur Herstellung von kosmetischen und/oder pharmazeutischen Produkten, beispielsweise für den Bereich der Haar- und Körperpflege.

Die genannten Pflegemittel, wie beispielsweise Haarshampoos, Duschbäder, Schaumbäder, Tagescremes, Nachtcremes, Pflegecremes, Nährcreme, Bodylotions, Salben und dergleichen, können als weitere Hilfs- und Zusatzstoffe Emulgatoren, kationische Polymere, Siliconverbindungen, Überfettungsmittel, Fette, Wachse, Stabilisatoren, biogene Wirkstoffe, Konservierungsmittel, Farb- und Duftstoffe enthalten.

Als **Emulgatoren bzw. Co-Emulgatoren** können nichtionogene, ampholytische und/ oder zwitterionische grenzflächenaktive Verbindungen verwendet werden, die sich durch eine lipophile, bevorzugt lineare, Alkyl- oder Alkenylgruppe und mindestens eine hydrophile Gruppe auszeichnen. Diese hydrophile Gruppe kann sowohl eine ionogene als auch eine nichtionogene Gruppe sein.

Nichtionogene Emulgatoren enthalten als hydrophile Gruppe z. B. eine Polyolgruppe, eine Polyalkylenglycolethergruppe oder eine Kombination aus Polyol- und Polyglycolethergruppe. Bevorzugt sind solche Mittel, die als **O/W-Emulgatoren** nichtionogene Tenside aus mindestens einer der folgenden Gruppen enthalten: (a1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe; (a2) C_{12/18}-Fettsäuremono- und -diester von Anlagerungsprodukten von 1 bis 30 Mol Ethylenoxid an Glycerin; (a3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte; (a4) Alkylmono- und -oligoglycoside mit 8 bis 22 Kohlenstoffatomen im Alkylrest und deren ethoxylierte Analoga und (a5) Anlagerungsprodukte von 15 bis 60 Mol Ethylenoxid an Ricinusöl und/oder gehärtetes Ricinusöl; (a6) Polyol- und insbesondere Polyglycerinester wie z.B. Polyglycerinpolyricinoleat oder Po1yg1ycerinpo1y-12-hydroxystearat. Ebenfalls geeignet sind Gemische von Verbindungen aus mehreren dieser Substanzklassen. Die Anlagerungsprodukte von Ethylenoxid und/oder von Propylenoxid an Fettalkohole, Fettsäuren, Alkylphenole, Glycerin-mono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, ent-spricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt. Besonders geeignete zwitterionische Tenside sind die sogenannten Betaine wie die N-Alkyl-N,N-dimethylammoniumglycinate, beispielsweise das Kokosalkyl-dimethylammoniumglycinat, N-Acyl-aminopropyl-N,N-dimethylammoniumglycinate, beispielsweise das Kokosacylaminopropyldimethylammoniumglycinat, und 2-Alkyl-3-carboxyl- methyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxy-ethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin.

Als **W/O-Emulgatoren** kommen in Betracht: (b1) Anlagerungsprodukte von 2 bis 15 Mol Ethylenoxid an Ricinusöl und/oder gehärtetes Ricinusöl; (b2) Partialester auf Basis linearer, verzweigter, ungesättigter bzw. gesättigter C_{12/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit) sowie Polyglucoside (z.B. Cellulose); (b3) Trialkylphosphate; (b4) Woll-wachsalkohole; (b5) Polysiloxan-Polyalkyl-Polyether-Copolymere bzw. entsprechende Derivate; (b6) Mischester aus Pentaerythrit, Fettsäuren, Citronensäure und Fettalkohol gemäß **DE-PS 11 65 574** sowie (b7) Polyalkylenglycole.

Geeignete **kationische Polymere** sind beispielsweise kationischen Cellulosederivate, kationischen Stärke, Copolymere von Diallylammoniumsalzen und Acrylamiden, quaternierte Vinylpyrrolidon/Vinylimidazol-Polymere wie z.B. Luviquat® (BASF AG, Ludwigshafen/FRG), Kondensationsprodukte von Polyglycolen und Aminen, quaternierte Weizen- oder Kollagenpolypeptide wie beispielsweise Lauryldimonium hydroxypropyl hydrolyzed collagen (Lamequat®L, Gluadin® WQ, Grünau/Illertissen), Polyethylenimin, kationische Siliconpolymere wie z.B. Amidomethicone oder Dow Corning, Dow Corning Co./US, Copolymere der Adipinsäure und Dimethylaminohydroxypropyldiethylentrimamin (Cartaretine®, Sandoz/ CH), Polyaminopolyamide wie z.B. beschrieben in der **FR-A 22 52 840** sowie deren vernetzte wasserlöslichen Polymere, kationische Chitinderivate wie beispielsweise quaterniertes Chitosan, gegebenenfalls mikrokristallin verteilt, kationischer Guar-Gum wie z.B. Jaguar® CBS, Jaguar® C-17, Jaguar® C-16 der Celanese/US bzw. Cosmedia Guar® C 261, quaternierte Ammoniumsalz-Polymere wie z.B. Mirapol® A-15, Mirapol® AD-1, Mirapol® AZ-1 der Miranol/US.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor- und/oder alkylmodifizierte Siliconverbindungen. Als **Überfettungsmittel** können Substanzen wie beispielsweise polyethoxylierte Lanolinderivate, Lecithinderivate, Polyolfettsäureester und Fettsäurealkanolamide verwendet werden, wobei die letzteren gleichzeitig als Schaumstabilisatoren dienen. Typische Beispiele für **Fette** sind Glyceride, als **Wachse** kommen u.a. Bienenwachs, Paraffinwachs oder Mikrowachse gegebenenfalls in Kombination mit hydrophilen Wachsen, z.B. Cetylstearylalkohol in Frage. Als **Stabilisatoren** können Metallsalze von Fettsäuren wie z.B. Magnesium-, Aluminium und/oder Zinkstearat eingesetzt werden. Unter **biogenen Wirkstoffen** sind beispielsweise Pflanzenextrakte und Vitaminkomplexe zu verstehen. Als **Konservierungsmittel** eignen sich beispielsweise Phenoxyethanol, Formaldehydlösung, Parabene, Pentandiol oder Sorbinsäure. Als **Perlglanzmittel** kommen beispielsweise Glycoldistearinsäureester wie Ethylenglycoldistearat, aber auch Fettsäuremonoglycolester in Betracht. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-%-bezogen auf die Mittel - betragen. Die Herstellung der Mittel kann in an sich bekannter Weise, d.h. beispielsweise durch Heiß-, Kalt-, Heiß-Heiß/Kalt- bzw. PIT-Emulgierung erfolgen. Hierbei handelt es sich um ein rein mechanisches Verfahren, eine chemische Reaktion findet nicht statt.

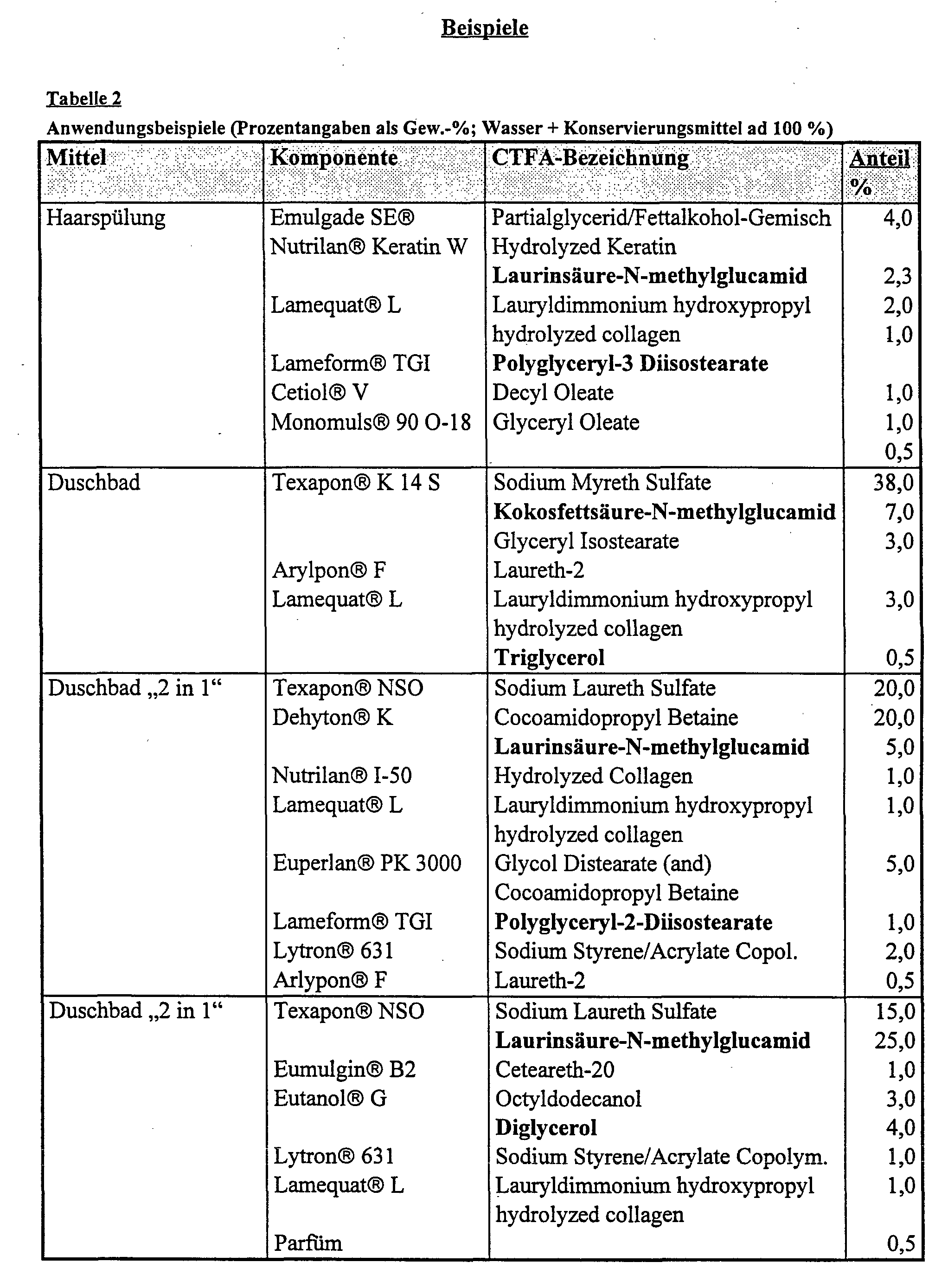

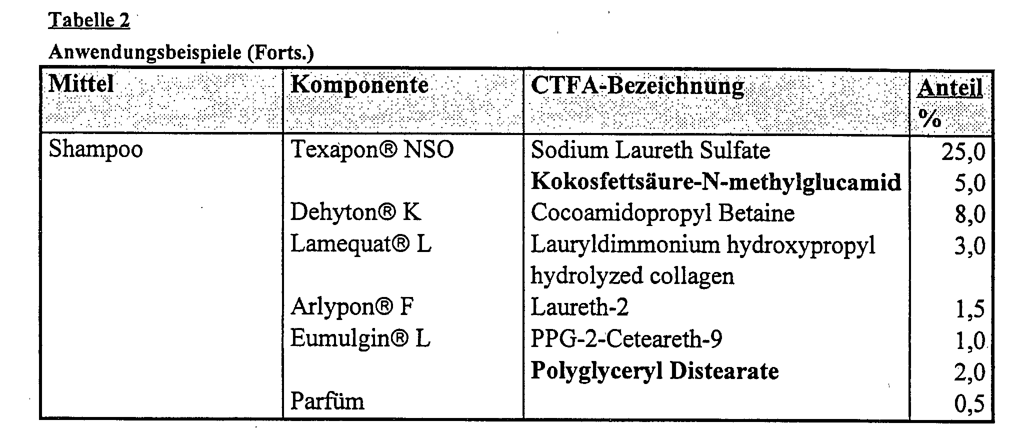

## Patentansprüche

1. Kosmetische und/oder pharmazeutische Zubereitungen, enthaltend
(a) Fettsäure-N-alkylpolyhydroxyalkylamide und
(b1) Oligoglycerine und/oder
(b2) Oligoglycerinfettsäureester.

2. Zubereitungen nach Anspruch 1, **dadurch gekennzeichnet, daß** sie Fettsäure-N-alkylpolyhydroxyalkylamide der Formel (I) enthalten, 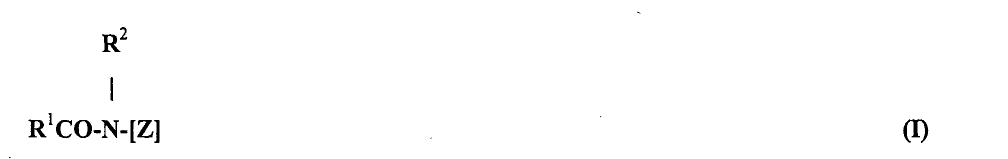 in der R¹CO für einen aliphatischen Acylrest mit 6 bis 22 Kohlenstoffatomen, R² für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

3. Zubereitungen nach den Ansprüchen 1 und 2, **dadurch gekennzeichnet, daß** sie Oligoglycerine mit einem mittleren Eigenkondensationsgrad im Bereich von 1,2 bis 5 enthalten.

4. Zubereitungen nach den Ansprüchen 1 bis 3, **dadurch gekennzeichnet, daß** sie Oligoglycerinfettsäureester enthalten, die sich von technischen Oligoglycerinen mit einem mittleren Eigenkondensationsgrad im Bereich von 1,2 bis 1,5 ableiten.

5. Zubereitungen nach den Ansprüchen 1 bis 4, **dadurch gekennzeichnet, daß** sie Oligoglycerinfettsäureester enthalten, die sich von Fettsäuren der Formel **(III)** ableiten.
**R**^{**3**}**CO-OH** (III)
in der R³CO für einen linearen oder verzweigten Acylrest mit 6 bis 22 Kohlenstoffatomen und 0 und/oder 1, 2 oder 3 Doppelbindungen steht.

6. Zubereitungen nach den Ansprüchen 1 bis 5, **dadurch gekennzeichnet, daß** sie Oligoglycerinfettsäureester enthalten, die einen mittleren Veresterungsgrad im Bereich von 1 bis 3 aufweisen.

7. Zubereitungen nach den Ansprüchen 1 bis 6, **dadurch gekennzeichnet, daß** sie weiterhin anionische, nichtionische, kationische und/oder amphotere bzw. zwitterionische Tenside enthalten.

8. Zubereitungen nach den Ansprüchen 1 bis 7, **dadurch gekennzeichnet, daß** sie weiterhin Ölkörper und/oder Emulgatoren enthalten.

9. Zubereitungen nach den Ansprüchen 1 bis 8, **dadurch gekennzeichnet, daß** sie
(a) 0,5 bis 70 Gew.-% Fettsäure-N-alkylpolyhydroxyalkylamide,
(b) 0,5 bis 40 Gew.-% Oligoglycerine und/oder Oligoglycerinfettsäureester,
(c) 0 bis 40 Gew.-% anionische, nichtionische, kationische und/oder amphotere bzw. zwitterionische Tenside,
(d) 0 bis 25 Gew.-% Ölkörper und
(e) 0 bis 25 Gew.-% Glycerin und/oder Wasser enthalten.

10. Verwendung der Zubereitungen nach Anspruch 1 als Rückfettungsmittel zur Herstellung von kosmetischen und/oder pharmazeutischen Produkten.

## Claims

1. Cosmetic and/or pharmaceutical compositions comprising
(a) fatty acid N-alkylpolyhydroxyalkylamides and
(b1) oligoglycerols and/or
(b2) oligoglycerol fatty acid esters.

2. Preparations according to Claim 1, **characterized in that** they comprise fatty acid N-alkylpolyhydroxyalkylamides of the formula (I) 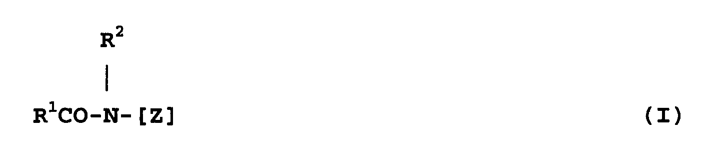 in which R¹CO is an aliphatic acyl radical having 6 to 22 carbon atoms, R² is hydrogen, an alkyl or hydroxyalkyl radical having 1 to 4 carbon atoms and [Z] is a linear or branched polyhydroxyalkyl radical having 3 to 12 carbon atoms and 3 to 10 hydroxyl groups.

3. Preparations according to Claims 1 and 2, **characterized in that** they comprise oligoglycerols with an average degree of self-condensation in the range from 1.2 to 5.

4. Preparations according to Claims 1 to 3, **characterized in that** they comprise oligoglycerol fatty acid esters derived from technical-grade oligoglycerols with an average degree of self-condensation in the range from 1.2 to 1.5.

5. Preparations according to Claims 1 to 4, **characterized in that** they comprise oligoglycerol fatty acid esters derived from fatty acids of the formula (III)
**R**^{**3**}**CO-OH** (III)
in which R³CO is a linear or branched acyl radical having 6 to 22 carbon atoms and 0 and/or 1, 2 or 3 double bonds.

6. Preparations according to Claims 1 to 5, **characterized in that** they comprise oligoglycerol fatty acid esters which have an average degree of esterification in the range from 1 to 3.

7. Preparations according to Claims 1 to 6, **characterized in that** they further comprise anionic, nonionic, cationic and/or amphoteric or zwitterionic surfactants.

8. Preparations according to Claims 1 to 7, **characterized in that** they further comprise oily substances and/or emulsifiers.

9. Preparations according to Claims 1 to 8, **characterized in that** they comprise
(a) 0.5 to 70% by weight of fatty acid N-alkylpolyhydroxyalkylamides,
(b) 0.5 to 40% by weight of oligoglycerols and/or oligoglycerol fatty acid esters,
(c) 0 to 40% by weight of anionic, nonionic, cationic and/or amphoteric or zwitterionic surfactants,
(d) 0 to 25% by weight of oily substances and
(e) 0 to 25% by weight of glycerol and/or water.

10. The use of the preparations according to Claim 1 as refatting agents for the preparation of cosmetic and/or pharmaceutical products.

## Revendications

1. Préparations consmétiques et/ou pharmaceutiques qui contiennent
a) des N-alkylpolyhydroxyalkylamides d'acides gras et
b1) des oligoglycérols et/ou
b2) des esters d'acide gras et d'oligoglycérols

2. Préparations selon la revendication 1,
**caractérisée en ce qu'**
elles renferment des N-alkylpolyhydroxyalkylamides d'acide gras de formule (I) 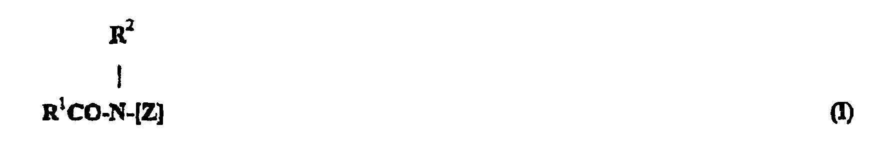 dans laquelle R¹CO représente un reste acyle aliphatique ayant de 6 à 22 atomes de carbone, R² représente l'hydrogène, un reste alkyle ou hydroxyalkyle ayant de 1 à 4 atomes de carbone et [Z] représente un reste polyhydroxyalkyle linéaire ou ramifié ayant de 3 à 12 atomes de carbone et de 3 à 10 groupes hydroxyle.

3. Préparations selon les revendications 1 et 2,
**caractérisées en ce qu'**
elles renferment des oligoglycérols ayant un degré moyen d'autocondensation dans la zone de 1, 2 à 5.

4. Préparations selon les revendications 1 à 3,
**caractérisées en ce qu'**
elles renferment des esters d'acide gras et d'oligoglycérol, qui dérivent d'oligoglycérols industriels ayant un degré moyen d'autocondensation dans la zone de 1,2 à 1,5.

5. Préparations selon les revendications 1 à 4,
**caractérisées en ce qu'**
elles renferment des esters d'acide gras et d'oligoglycérols qui dérivent d'acides gras de formule (III)
R³CO-OH (III)
dans laquelle R³CO représente un reste acyle, linéaire ou ramifié ayant de 6 à 22 atomes de carbone et 0 et/ou 1, 2 ou 3 double liaisons.

6. Préparations selon les revendications 1 à 5,
**caractérisées en ce qu'**
elles renferment des esters d'acide gras et d'oligoglycérols qui possèdent un degré moyen d'esterification dans la zone de 1 à 3.

7. Préparations selon les revendications 1 à 6,
**caractérisées en ce qu'**
elles renferment en outre des agents tensioactifs anioniques, non-ioniques, cationiques et/ou amphotères ou zwitterioniques.

8. Préparations selon les revendications 1 à 7,
**caractérisées en ce qu'**
elles renferment en outre des composés huileux et/ou des agents émulsionnants.

9. Préparations selon les revendications 1 à 8,
**caractérisées en ce qu'**
elles renferment
a) de 0,5 à 70 % en poids de N-alkylpolyhydroxyalkylamides d'acides gras
b) de 0,5 à 40 % en poids d'oligoglycérols et/ou d'esters d'acide gras et d'oligoglycérol
c) de 0 à 40 % en poids d'agents tensio actifs anioniques, non ioniques cationiques, et/ou amphotères ou zwitterioniques
d) de 0 à 25 % en poids de composés huileux et
e) de 0 à 25 % en poids de glycérol et/ou d'eau.

10. Utilisation des préparations selon la revendication 1,
comme agent de regraissage en vue de la préparation de produits cosmétiques et/ou pharmaceutiques.
